# EUROPÄISCHE PATENTANMELDUNG

(11) **EP 0 743 069 A1**
(43) Veröffentlichungstag der Anmeldung: **20.11.1996**
(21) Anmeldenummer: 95107654.6
(22) Anmeldetag: 19.05.1995
(51) Int. Cl.: A61K 38/06, A61K 38/22

(54) **Pharmazeutische Zubereitungen zur Behandlung von Migräne**

(71) Anmelder: Ludewigs, Carl Heinz, D-21244 Buchholz (DE)
(72) Erfinder: Ludewigs, Carl Heinz, D-21244 Buchholz (DE)

(57) **Zusammenfassung**

Gegenstand der Erfindung sind pharmazeutische Zubereitungen zur Behebung der Hemikranie(Migräne) beim Menschen. Dadurch gekennzeichnet, daß sie Schilddrüsenhormone in ihrer natürlichen Zusammensetzung, in Form von L-Thyroxin, TRH oder andere entsprechende Wirkstoffe enthalten.

Gegenstand der Erfindung ist weiter ein Verfahren zur Behebung der Hemikranie(Migräne) beim Menschen, durch Verabreichung der obengenannten Wirkstoffe.

Die bisher bekannten Therapien verwenden übliche Schmerzmittel, bei denen die Nachteile einer Dauerbehandlung bekannt sind. Neuerdings mit Ergotamintartrat und in neuster Zeit mit Sumatriptan wird gezielt in den Ablauf eines jeweiligen Migräneanfalls eingegriffen.

Das hier neu vorgestellte Verfahren zur Behebung der Migräne, führt zu einer Umstimmung des Organismus. Acht Wochen nach Beginn der Therapie besteht in der Regel Migränefreiheit.

## Beschreibung

Gegenstand der Erfindung sind pharmazeutische Zubereitungen, die Schilddrüsenhormone in ihrer natürlichen Zusammensetzung oder in reiner Form als L-Thyroxin enthalten. Ferner pharmazeutische Zubereitungen, die - wie etwa die Hypophyse anregende Hormone (TRH), welche die Ausschüttung von Thyreotropin bewirken, das seinerseits eine Erhöhung des Schilddrüsenhormonspiegels im Körper bewirkt - körpereigene Reaktionen ausnützen.

Gegenstand der Erfindung ist weiter ein Verfahren zur Behebung der Hemikranie(Migräne) beim Menschen, dadurch gekennzeichnet, daß oben genannte pharmazeutische Zubereitungen peroral verabreicht werden.

Schilddrüsenhormone sind in Bezug auf ihre Verwendung in der inneren Medizin und speziell in der Endokrinologie ausführlich in der Fachliteratur beschrieben worden. Die Migräne-Therapie ist bis in die neuere Zeit mit üblichen Schmerzmitteln durchgeführt worden. Ergotamintartrat ist eines der neueren Mittel, mit denen mar versuchte, der Migräne besser Herr zu werden. Das neueste Mittel, Sumatriptan, muß ebenfalls bei einem akuten Migräneanfall eingesetzt werden.

Als grundlegende Verbesserung der Migräne-Therapie hat sich der Einsatz von Schilddrüsenhormonen erwiesen, die in geringen Mengen verabreicht, in etwa 8 Wochen zur Migränefreiheit führten. Vermutlich wird durch diese Therapie eine Umstimmung im Organismus erzielt, die durch Fortsetzung der Hormontherapie erhalten werden konnte.

Verabreichung von L-Thyroxin wurde folgendermaßen durchgeführt: 1. bis 4. Woche 2x25γ pro Woche, 5. bis 8. Woche 2x50γ pro Woche; dann als erhaltungsdosis 25-50γ 2x pro Woche. (1000γ = 1mg)

## Patentansprüche

1. Pharmazeutische Zubereitungen zur Behebung der Hemikranie (Migräne) beim Menschen, bestehend aus Schilddrüsenhormonen und üblichen, pharmazeutisch unbedenklichen Zusatz- und Trägerstoffen.

2. Pharmazeutische Zubereitungen zur Behebung der Hemikranie (Migräne) beim Menschen, bestehend aus Thyreotropin stimulierenden Hormonen (TRH), die über die Hypophyse die Ausschüttung von Schilddrüsenhormonen bewirken; außerdem andere entsprechend reagierende Wirkstoffe.
